# EUROPÄISCHE PATENTSCHRIFT

(11) **EP 2 331 596 B1**
(45) Veröffentlichungstag und Bekanntmachung des Hinweises auf die Patenterteilung: **22.02.2012**
(21) Anmeldenummer: 09778050.6
(22) Anmeldetag: 22.08.2009
(51) Int. Cl.: C08G 18/12, C08G 18/44, C08G 64/02, C08J 3/07, C09D 175/08, A61L 31/10

(54) **TCD-BASIERTE HYDROPHILE POLYURETHANDISPERSIONEN**
TCD BASED HYDROPHILIC POLYURETHANE DISPERSIONS
DISPERSIONS DE POLYURÉTHANE HYDROPHILES À BASE DE TCD

(30) Priorität: 04.09.2008 EP 08015573
(43) Veröffentlichungstag der Anmeldung: 15.06.2011
(73) Patentinhaber: Bayer MaterialScience AG, 51368 Leverkusen (DE)
(72) Erfinder: KÖCHER, Jürgen, 40764 Langenfeld (DE); WAMPRECHT, Christian, 41472 Neuss (DE)
(86) Internationale Anmeldenummer: PCT/EP2009/006101
(87) Internationale Veröffentlichungsnummer: WO 2010/025840

(56) Entgegenhaltungen:
- WO-A-2006/093355
- WO-A-2006/109816
- US-A- 5 756 632
- US-A1- 2005 182 188

## Beschreibung

Die vorliegende Erfindung betrifft neuartige wässrige Polyurethandispersionen, welche zur Herstellung von hydrophilen Beschichtungen verwendet werden können.

Die Benutzung von medizinischen Geräten, beispielsweise von Kathetern, kann durch die Ausrüstung mit hydrophilen Oberflächen stark verbessert werden. Das Einsetzen und Verschieben von Urin- oder Blutgefäßkathetern wird dadurch einfacher, dass hydrophile Oberflächen im Kontakt mit Blut oder Urin einen Wasserfilm adsorbieren. Hierdurch wird die Reibung der Katheteroberfläche gegenüber den Gefäßwänden verringert, so dass sich der Katheter leichter einsetzen und bewegen lässt. Auch eine direkte Wässerung der Geräte vor dem Eingriff kann vorgenommen werden, um durch die Bildung eines homogenen Wasserfilms die Reibung zu vermindern. Die betroffenen Patienten haben weniger Schmerzen, und das Risiko von Verletzungen der Gefäßwände wird dadurch reduziert. Darüber hinaus besteht bei der Anwendung von Kathetern im Blutkontakt immer die Gefahr, dass sich Blutgerinnsel bilden. In diesem Kontext werden im Allgemeinen hydrophile Beschichtungen als hilfreich für antithrombogene Beschichtungen angesehen.

Zur Herstellung von entsprechenden Oberflächen eignen sich grundsätzlich Polyurethanbeschichtungen, die ausgehend von Lösungen oder Dispersionen entsprechender Polyurethane hergestellt werden.

So beschreibt die US 5,589,563 die Verwendung von Beschichtungen mit oberflächenmodifizierten Endgruppen für im Bereich der Biomedizin verwendete Polymere, welche auch zur Beschichtung von medizinischen Geräten verwendet werden können. Die resultierenden Beschichtungen werden ausgehend von Lösungen oder Dispersionen hergestellt und die polymeren Beschichtungen umfassen unterschiedliche Endgruppen, welche ausgewählt werden aus Aminen, fluorierten Alkanolen, Polydimethylsiloxanen und aminterminierten Polyethylenoxiden. Diese Polymere weisen jedoch als Beschichtung für medizinische Geräte keine zufriedenstellenden Eigenschaften, insbesondere im Hinblick auf die erforderliche Hydrophilie, auf.

Die DE 199 14 882 A1 betrifft Polyurethane, Polyurethan-Harnstoffe bzw. Polyharnstoffe in dispergierter bzw. gelöster Form, die aufgebaut werden aus
a) mindestens einer Polyolkomponente,
b) mindestens einer Di-, Tri- und/oder Polyisocyanatkomponente,
c) mindestens einer hydrophilen, nichtionischen bzw. potentiell ionischen Aufbaukomponente, bestehend aus Verbindungen mit mindestens einer gegenüber Isocyanatgruppen reaktiven Gruppe und mindestens einer hydrophilen Polyetherkette und/oder aus Verbindungen mit mindestens einer, gegebenenfalls mindestens teilweise neutralisiert vorliegenden, zur Salzbildung fähigen Gruppe und mindestens einer gegenüber Isocyanatgruppen reaktiven Gruppe,
d) mindestens einer von a) bis c) verschiedenen Aufbaukomponente des Molekulargewichtsbereiches 32 bis 500 mit mindestens einer gegenüber Isocyanatgruppen reaktiven Gruppe und
e) mindestens eines monofunktionellen Blockierungsmittels. Die damit zwingend ein monofunktionelles Blockierungsmittel aufweisenden Polymerdispersionen werden in Schlichten verwendet.

Die DE 199 14 885 A1 betrifft Dispersionen auf Basis von Polyurethanen, PolyurethanPolyharnstoffen bzw. Polyharnstoffen die bevorzugt Umsetzungsprodukte darstellen von
a) mindestens einer Polyolkomponente,
b) mindestens einer Di-, Tri- und/oder Polyisocyanatkomponente,
c) gegebenenfalls mindestens einer (potentiell) ionischen Aufbaukomponente, bestehend aus Verbindungen mit mindestens einer gegenüber NCO-Gruppen reaktiven Gruppe und mindestens einer, gegebenenfalls mindestens teilweise neutralisiert vorliegenden, zur Salzbildung fähigen Gruppe,
d) gegebenfalls mindestens einer nichtionisch hydrophilen Aufbaukomponente, bestehend aus im Sinne der Isocyanatadditionsreaktion mono- bis tetrafunktionellen, mindestens eine hydrophile Polyetherkette aufweisenden Verbindungen,
e) gegebenenfalls mindestens einer von a) bis d) verschiedenen Aufbaukomponente des Molekulargewichtsbereiches 32 bis 2500 mit gegenüber Isocyanatgruppen reaktiven Gruppen und
f) 0,1 bis 15 Gew.-% mindestens eines monofunktionellen Blockienngsmittels, welches zu mindestens 50% aus Dimethylpyrazol besteht,
wobei die Summe aus a) bis f) 100% beträgt und wobei entweder c) oder d) nicht 0 sein kann und in solcher Menge eingesetzt werden, dass eine stabile Dispersion entsteht. Die Dispersionen werden unter anderem zur Beschichtung mineralischer Untergründe, zur Lackierung und Versiegelung von Holz und Holzwerkstoffen, zur Lackierung und Beschichtung metallischer Oberflächen, Lackieren und Beschichten von Kunststoffen sowie die Beschichtung von Textilien und Leder verwendet.

Diese aus dem Stand der Technik bekannten Polyurethanharnstoff-Dispersionen werden nicht für medizinische Zwecke, d.h. zum Beschichten von medizinischen Geräten, verwendet. Darüber hinaus weisen die bisher bekannten Polyurethanharnstoffbeschichtungen häufig dahingehend Nachteile auf, dass sie für eine Anwendung als Beschichtung von medizinischen Geräten nicht ausreichend hydrophil ausgebildet sind.

In diesem Kontext empfiehlt die US 5,589,563 oberflächenmodifizierte Endgruppen für biomedizinisch Polymere, welche zur Beschichtung von medizinischen Geräten verwendet werden können. Diese Polymere umfassen unterschiedliche Endgruppen, welche ausgewählt werden aus Aminen, fluorierten Alkanolen, Polydimethylsiloxanen und aminterminierten Polyethylenoxiden. Diese Polymere weisen jedoch als Beschichtung für medizinische Geräte ebenfalls keine zufrieden stellenden Eigenschaften, insbesondere im Hinblick auf die erforderliche Hydrophilie, auf.

Aus der nicht vorveröffentlichten Europäischen Anmeldung Nr. 08153053.7 sind nun wässrige Dispersionen bekannt, welche sich hervorragend zur Herstellung hydrophiler Beschichtungen einsetzen lassen.

Es wurde nun gefunden, dass sich die mechanischen Eigenschaften solcher Beschichtungen durch den Einsatz spezieller Polycarbonatdiole noch verbessern lässt.

Gegenstand der Erfindung sind daher Polyurethanharnstoff-Dispersionen umfassend Polyurethanharnstoffe, die
(1) mit wenigstens einer polyethylenoxid- und polypropylenoxidbasierten Copolymereinheit terminiert sind, und
(2) Polycarbonatpolyol-basierte Einheiten gemäß Formel (1) umfassen

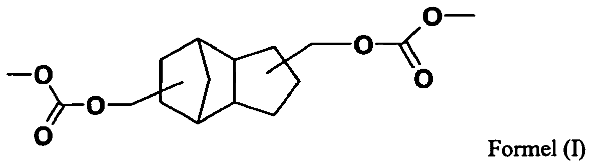

Erfindungsgemäß wurde herausgefunden, dass sich Zusammensetzungen aus diesen spezifischen Polyurethanharnstoffen hervorragend als hydrophile Beschichtungen unter anderem für medizinische Geräte eignen, diese mit einer hervorragenden gleitfähigen Beschichtung versehen und gleichzeitig die Gefahr der Bildung von Blutgerinnseln während der Behandlung mit dem medizinischen Gerät reduzieren.

Polyurethanharnstoffe im Sinne der vorliegenden Erfindung sind polymere Verbindungen, die
a) mindestens zwei Urethangruppen enthaltende Wiederholungseinheiten der folgenden allgemeinen Struktur 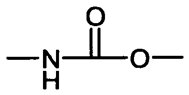 und
b) mindestens eine Harnstoffgruppen enthaltende Wiederholungseinheit 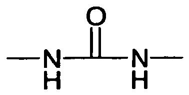
aufweisen.

Die erfindungsgemäßen Dispersionen basieren auf Polyurethanhamstoffen, welche im Wesentlichen keine ionische Modifizierung aufweisen. Hierunter wird im Rahmen der vorliegenden Erfindung verstanden, dass die erfindungsgemäß zu verwendenden Polyurethanharnstoffe im Wesentlichen keine ionischen Gruppen, wie insbesondere keine Sulfonat-, Carboxylat-, Phosphat- und Phosphonatgruppen, aufweisen.

Unter dem Begriff "im Wesentlichen keine ionische Modifizierung" wird im Rahmen der vorliegenden Erfindung verstanden, dass eine ionische Modifizierung höchstens in einem Anteil von 2,50 Gew.-%, vorzugsweise höchstens 2,00 Gew.-%, insbesondere höchstens 1,50 Gew.-%, besonders bevorzugt höchstens 1,00 Gew.-%, speziell höchstens 0,50 Gew.-%, vorliegt, wobei es am bevorzugtesten ist, wenn überhaupt keine ionische Modifizierung des erfindungsgemäß vorgesehenen Polyurethanharnstoffs vorliegt.

Die erfindungswesentlichen Polyurethanharnstoffe der vorstehend genannten Art sind bevorzugt im Wesentlichen lineare Moleküle, können jedoch auch verzweigt sein, was jedoch weniger bevorzugt ist. Unter im Wesentlichen linearen Molekülen versteht man im Rahmen der vorliegenden Erfindung leicht anvemetzte Systeme, wobei die zugrundeliegende Polycarbonatpolyolkomponente eine mittlere Hydroxylfunktionalität von bevorzugt 1,7 bis 2,3, besonders bevorzugt 1,8 bis 2,2, ganz besonders bevorzugt 1,9 bis 2,1, aufweist. Derartige Systeme lassen sich noch in einem ausreichenden Maß dispergieren.

Das zahlenmittlere Molekulargewicht der erfindungswesentlichen Polyurethanharnstoffe beträgt bevorzugt 1000 bis 200000 g/mol, besonders bevorzugt von 5000 bis 100000 g/mol. Dabei wird das zahlenmittlere Molekülgewicht gegen Polystyrol als Standard in Dimethylacetamid bei 30 °C gemessen.

Die erfindungswesentlichen Polyurethanharnstoffe werden durch Umsetzung von Aufbaukomponenten hergestellt, die mindestens eine Polycarbonatpolyolkomponente a), mindestens eine Polyisocyanatkomponente b), mindestens eine Polyoxyalkylenetherkomponente c), mindestens eine Diamin- und/oder Aminoalkoholkomponente d) und gegebenenfalls eine weitere Polyolkomponente umfassen.

Durch Dispergierung in Wasser werden die erfindungsgemäßen Dispersionen erhalten.

Daher ist ein weiterer Gegenstand der vorliegenden Erfindung ein Verfahren zur Herstellung der Polyurethanharnstoff-Dispersionen, bei dem eine Polycarbonatpolyolkomponente a), mindestens eine Polyisocyanatkomponente b), mindestens eine Polyoxyalkylenetherkomponente c), mindestens eine Diamin- und/oder Aminoalkoholkomponente d) und gegebenenfalls eine weitere Polyolkomponente miteinander umgesetzt werden und eine Dispergierung in Wasser erfolgt.

Komponente a) umfasst wenigstens ein Polycarbonatpolyol a1), welches durch Umsetzung von Kohlensäurederivaten, wie Diphenylcarbonat, Dimethylcarbonat oder Phosgen mit difunktionellen Alkoholen der Formel (II) 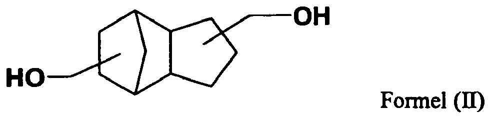 erhalten wird.

Zur Herstellung wird in einem Druckreaktor bei erhöhter Temperatur TCD Alkohol DM [3(4),8(9)-Bis(hydroxymethyl)-tricyclo(5.2.1.0/2.6)decan/ Tricyclodecandimethanol] mit Diphenylcarbonat, Dimethylcarbonat oder Phosgen umgesetzt. Bevorzugt ist die Umsetzung mit Dimethylcarbonat. Im Falle der Verwendung von Dimethylcarbonat wird das Spaltprodukt Methanol im Gemisch mit überzähligem Dimethylcarbonat per Destillation entfernt.

Diese Polycarbonatpolyole a1) auf Basis von Diolen der Formel (II) haben durch die OH-Zahl bestimmte Molekulargewichte von vorzugsweise 200 bis 10000 g/mol, besonders bevorzugt 300 bis 8000 g/mol und ganz besonders bevorzugt 400 bis 6000 g/mol.

Bevorzugt ist Komponente a) eine Mischung aus vorgenannten Polycarbonatpolyolen a1) auf Basis von Diolen der Formel (II) und weiteren Polycarbonatpolyolen a2).

Solche weiteren Polycarbonatpolyole a2) haben bevorzugt mittlere Hydroxylfunktionalitäten von 1,7 bis 2,3, besonders bevorzugt von 1,8 bis 2,2, ganz besonders bevorzugt von 1,9 bis 2,1.

Ferner weisen die Polycarbonatpolyole a2) durch die OH-Zahl bestimmte Molekulargewichte von vorzugsweise 400 bis 6000 g/mol, besonders bevorzugt 500 bis 5000 g/mol, insbesondere von 600 bis 3000 g/mol auf, die beispielsweise durch Reaktion von Kohlensäurederivaten, wie Diphenylcarbonat, Dimethylcarbonat oder Phosgen, mit Polyolen, vorzugsweise Diolen, erhältlich sind. Als derartige Diole kommen beispielsweise Ethylenglykol, 1,2- und 1,3-Propandiol, 1,3- und 1,4-Butandiol, 1,6-Hexandiol, 1,8-Octandiol, Neoperitylglykol, 1,4-Bishydroxymethylcyclohexan, 2-Methyl-1,3-propandiol, 2,2,4-Trimethylpentan-1,3-diol, Di-, Tri- oder Tetraethylenglykol, Dipropylenglykol, Polypropylenglykole, Dibutylenglykol, Polybutylenglykole, Bisphenol A, Tetrabrombisphenol A aber auch Lacton-modifizierte Diole in Frage.

Diese Polycarbonatpolyole a2) enthalten bevorzugt 40 bis 100 Gew.-% Hexandiol, bevorzugt 1,6-Hexandiol und/oder Hexandiol-Derivate, vorzugsweise solche, die neben endständigen OH-Gruppen Ether- oder Estergruppen aufweisen, z.B. Produkte, die durch Umsetzung von 1 Mol Hexandiol mit mindestens 1 Mol, bevorzugt 1 bis 2 Mol Caprolacton oder durch Veretherung von Hexandiol mit sich selbst zum Di- oder Trihexylenglykol erhalten wurden, als Aufbaukomponenten. Auch Polyether-Polycarbonatdiole können eingesetzt werden. Die Hydroxylpolycarbonate sollten im wesentlichen linear sein. Sie können jedoch gegebenenfalls durch den Einbau polyfunktioneller Komponenten, insbesondere niedermolekularer Polyole, leicht verzweigt werden. Hierzu eignen sich beispielsweise Glycerin, Hexantriol-1,2,6, Butantriol-1,2,4, Trimethylolpropan, Pentaerythrit, Chinit, Mannit, Sorbit, Methylglykosid oder 1,3,4,6-Dianhydrohexite. Bevorzugt sind solche Polycarbonate a2) auf Basis von Hexandiol-1,6, sowie modifizierend wirkenden Co-Diolen wie z. B. Butandiol-1,4 oder auch von ε-Caprolacton. Weitere bevorzugte Polycarbonatdiole a2) sind solche auf Basis von Mischungen aus Hexandiol-1,6 und Butandiol-1,4.

In einer bevorzugten Ausführungsform wird in a) eine Mischung eingesetzt aus den Polycarbonatpolyolen a1) und solchen Polycarbonatpolyolen a2) auf Basis von Hexandiol-1,6, Butandiol-1,4 oder deren Mischungen.

Im Fall von Mischungen der Bestandteile a1) und a2) beträgt der Anteil von a1) an der Mischung bevorzugt mindestens 5 mol%, besonders bevorzugt mindestens 10 mol%, bezogen auf die gesamte Molmenge an Polycarbonat.

Die erfindungswesentlichen Polyurethanharnstoffe weisen ferner Einheiten auf, welche auf mindestens ein Polyisocyanat als Aufbaukomponente b) zurückgehen.

Als Polyisocyanate b) können alle dem Fachmann bekannten aromatischen, araliphatischen, aliphatischen und cycloaliphatischen Isocyanate einer mittleren NCO-Funktionalität ≥ 1, bevorzugt ≥ 2 einzeln oder in beliebigen Mischungen untereinander eingesetzt werden, wobei es unerheblich ist, ob diese nach Phosgen- oder phosgen-freien Verfahren hergestellt wurden. Diese können auch Iminooxadiazindion-, Isocyanurat-, Uretdion-, Urethan-, Allophanat-, Biuret-, Harnstoff-, Oxadiazintrion, Oxazolidinon-, Acylharnstoff- und/oder Carbodiimid-Strukturen aufweisen. Die Polyisocyanate können einzeln oder in beliebigen Mischungen untereinander eingesetzt werden.

Bevorzugt werden Isocyanate aus der Reihe der aliphatischen oder cycloaliphatischen Vertreter eingesetzt, wobei diese ein Kohlenstoffgrundgerüst (ohne die enthaltenen NCO-Gruppen) von 3 bis 30, bevorzugt 4 bis 20 Kohlenstoffatomen aufweisen.

Besonders bevorzugte Verbindungen der Komponente b) entsprechen der vorstehend genannten Art mit aliphatisch und/oder cycloaliphatisch gebundene NCO-Gruppen wie beispielsweise Bis-(isocyanatoalkyl)ether, Bis- und Tris-(isocyanatoalkyl)benzole, -toluole, sowie -xylole, Propandiisocyanate, Butandiisocyanate, Pentandiisocyanate, Hexandiisocyanate (z.B. Hexamethylendiisocyanat, HDI), Heptandiisocyanate, Octandiisocyanate, Nonandiisocyanate (z.B. Trimethyl-HDI (TMDI) in der Regel als Gemisch der 2,4,4- und 2,2,4-Isomeren), Nonantriisocyanate (z.B. 4-Isocyanatomethyl-1,8-octandiisocyanat), Dekandiisocyanate, Dekantriisocyanate, Undekandiisocyanate, Undekantriisocyanate, Dodecandiisocyanate, Dodecantriisocyanate, 1,3- sowie 1,4-Bis-(isocyanatomethyl)cyclohexane (H₆XDI), 3-Isocyanatomethyl-3,5,5-trimethylcyclohexylisocyanat (Isophorondiisocyanat, IPDI), Bis-(4-isocyanatocyclohexyl)methan (H₁₂MDI) oder Bis-(isocyanatomethyl)norbornan (NBDI).

Ganz besonders bevorzugte Verbindungen der Komponente b) sind Hexamethylendiisocyanat (HDI), Trimethyl-HDI (TMDI), 2-Methylpentan-1,5-diisocyanat (MPDI), Isophorondiisocyanat (IPDI), 1,3- sowie 1,4-Bis(isocyanatomethyl)cyclohexan (H₆XDI), Bis(isocyanatomethyl)norbornan (NBDI), 3(4)-Isocyanatomethyl-1-methyl-cyclohexylisocyanat (IMCI) und/oder 4,4'-Bis-(isocyanatocyclohexyl)methan (H₁₂MDI) oder Gemische dieser Isocyanate. Weitere Beispiele sind Derivate aus den vorstehenden Diisocyanaten mit Uretdion-, Isocyanurat-, Urethan-, Allophanat-, Biuret-, Iminooxadiazindion- und/oder Oxadiazintrionstruktur mit mehr als zwei NCO-Gruppen.

Die Menge an Bestandteil b) bei der Herstellung der erfindungswesentlichen Polyurethanharnstoffe beträgt vorzugsweise 1,0 bis 3,5 mol, besonders bevorzugt 1,0 bis 3,3 mol, insbesondere 1,0 bis 3,0 mol, jeweils bezogen auf die Menge der Verbindungen der Komponente a).

Der in der vorliegenden Erfindung verwendete Polyurethanharnstoff weist Einheiten auf, welche auf ein Copolymer aus Polyethylenoxid und Polypropylenoxid als Aufbaukomponente c) zurückgehen. Diese Copolymereinheiten liegen als Endgruppen in dem Polyurethanharnstoff vor und bewirken eine besonders vorteilhafte Hydrophilierung.

Solche nichtionisch hydrophilierenden Verbindungen c) sind beispielsweise einwertige, im statistischen Mittel 5 bis 70, bevorzugt 7 bis 55 Ethylenoxideinheiten pro Molekül aufweisende Polyalkylenoxidpolyetheralkohole, wie sie in an sich bekannter Weise durch Alkoxylierung geeigneter Startermoleküle zugänglich sind (z.B. in Ullmanns Enzyklopädie der technischen Chemie, 4. Auflage, Band 19, Verlag Chemie, Weinheim S. 31-38).

Geeignete Startermoleküle sind beispielsweise gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole. Besonders bevorzugt wird Diethylenglykolmonobutylether als Startermolekül verwendet.

Die Alkylenoxide Ethylenoxid und Propylenoxid können in beliebiger Reihenfolge oder auch im Gemisch bei der Alkoxylierungsreaktion eingesetzt werden.

Bei den Polyalkylenoxidpolyetheralkoholen handelt es sich um gemischte Polyalkylenoxidpolyether aus Ethylenoxid und Propylenoxid, deren Alkylenoxideinheiten vorzugsweise zu mindestens 30 mol%, besonders bevorzugt zu mindestens 40 mol-% aus Ethylenoxideinheiten bestehen. Bevorzugte nichtionische Verbindungen sind monofunktionelle gemischte Polyalkylenoxidpolyether, die mindestens 40 mol-% Ethylenoxid- und maximal 60 mol-% Propylenoxideinheiten aufweisen.

Das mittlere Molgewicht des Polyoxyalkylenethers beträgt vorzugsweise 500 g/mol bis 5000 g/mol, besonders bevorzugt 1000 g/mol bis 4000 g/mol, insbesondere 1000 bis 3000 g/mol.

Die Menge an Bestandteil c) bei der Herstellung der erfindungswesentlichen Polyurethanharnstoffe beträgt vorzugsweise 0,01 bis 0,5 mol, besonders bevorzugt 0,02 bis 0,4 mol, insbesondere 0,04 bis 0,3 mol, jeweils bezogen auf die Menge der Verbindungen der Komponente a).

Erfindungsgemäß konnte gezeigt werden, dass sich die Polyurethanharnstoffe mit Endgruppen, die auf gemischten Polyoxyalkylenether aus Polyethylenoxid und Polypropylenoxid basieren, insbesondere dazu eignen, Beschichtungen mit einer hohen Hydrophilie zu erzeugen.

Die erfindungswesentlichen Polyurethanharnstoffe weisen Einheiten auf, welche auf mindestens ein Diamin oder ein Aminoalkohol als Aufbaukomponente zurückgehen und als sogenannte Kettenverlängerer d) dienen.

Solche Kettenverlängerer sind beispielsweise Di- oder Polyamine sowie Hydrazide, z.B. Hydrazin, Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 1,3- und 1,4-Xylylendiamin, α,α,α',α'-Tetramethyl-1,3- und -1,4-xylylendiamin und 4,4'-Diaminodicyclohexylmethan, Dimethylethylendiamin, Hydrazin, A-dipinsäuredihydrazid, 1,4-Bis(aminomethyl)cyclohexan, 4,4'-Diamino-3,3'-dimethyldicyclohexyl-methan und andere (C₁ - C₄)-Di- und Tetraalkyldicyclo-hexylmethane, z. B. 4,4'-Diamino-3,5-diethyl-3',5'-diisopropyldicyclohexylinethan.

Als Diamine oder Aminoalkohole kommen im Allgemeinen niedermolekulare Diamine oder Aminoalkohole in Betracht, die aktiven Wasserstoff mit gegenüber NCO-Gruppen unterschiedlicher Reaktivität enthalten, wie Verbindungen, die neben einer primären Aminogruppe auch sekundäre Aminogruppen oder neben einer Aminogruppe (primär oder sekundär) auch OH-Gruppen aufweisen. Beispiele hierfür sind primäre und sekundäre Amine, wie 3-Amino-1-Methylaminopropan, 3-Amino-1-Ethylaminopropan, 3-Amino-1-cyclohexylaminopropan, 3-Amino-1- Methylaminobutan, weiterhin Aminoalkohole, wie N-Aminoethylethanolamin, Ethanolamin, 3-Aminopropanol, Neopentanolamin und besonders bevorzugt Diethanolamin.

Der Bestandteil d) der erfindungswesentlichen Polyurethanharnstoffe kann bei deren Herstellung als Kettenverlängerer eingesetzt werden.

Die Menge an Bestandteil d) bei der Herstellung der erfindungswesentlichen Polyurethanharnstoffe beträgt vorzugsweise 0,1 bis 1,5 mol, besonders bevorzugt 0,2 bis 1,3 mol, insbesondere 0,3 bis 1,2 mol, jeweils bezogen auf die Menge der Verbindungen der Komponente a).

In einer weiteren Ausführungsform umfassen die erfindungswesentlichen Polyurethanharnstoffe zusätzliche Einheiten, welche auf mindestens ein weiteres Polyol als Aufbaukomponente zurückgehen.

Die zum Aufbau der Polyurethanharnstoffe eingesetzten weiteren niedermolekularen Polyole e) bewirken in der Regel eine Versteifung und/oder eine Verzweigung der Polymerkette. Das Molekulargewicht beträgt vorzugsweise 62 bis 500 g/mol, besonders bevorzugt 62 bis 400 g/mol, insbesondere 62 bis 200 g/mol.

Geeignete Polyole können aliphatische, alicyclische oder aromatische Gruppen enthalten. Genannt seien hier beispielsweise die niedermolekularen Polyole mit bis zu etwa 20 Kohlenstoffatomen je Molekül, wie z. B. Ethyenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), sowie Trimethylolpropan, Glycerin oder Pentaerythrit und Mischungen dieser und gegebenenfalls auch weiterer niedermolekularer Polyole. Auch Esterdiole wie z.B. α-Hydroxybutyl-ε-hydroxycapronsäureester, ω-Hydroxyhexyl-γ-hydroxybuttersäure-ester, Adipinsäure-(ß-hydroxyethyl)ester oder Terephthalsäure-bis(ß-hydroxyethyl)-ester können verwendet werden.

Die Menge an Bestandteil e) bei der Herstellung der erfindungswesentlichen Polyurethanharnstoffe beträgt, falls vorhanden, vorzugsweise 0,05 bis 1,0 mol, besonders bevorzugt 0,05 bis 0,5 mol, insbesondere 0,1 bis 0,5 mol, jeweils bezogen auf die Menge der Verbindungen der Komponente a).

Die Umsetzung der isocyanathaltigen Komponente b) mit den hydroxy- oder aminfunktionellen Verbindungen a), c), d) und gegebenenfalls e) erfolgt üblicherweise unter Einhaltung eines leichten NCO-Überschusses gegenüber den reaktiven Hydroxy- oder Aminverbindungen. Diese Reste müssen abgebaut oder blockiert werden, damit nicht eine Reaktion mit großen Polymerketten stattfindet. Eine solche Reaktion führt zur dreidimensionalen Vernetzung und Vergelung des Ansatzes, so dass eine Weiterverarbeitung nicht mehr möglich ist.

Üblicherweise werden die überschüssigen Isocyanatgruppen jedoch während des Dispergierschrittes durch das Dispergierwasser hydrolysiert und abgebaut.

Wurde bei der Herstellung der erfindungswesentlichen Polyurethanharnstoffe der Restisocyanatgehalt blockiert, weisen diese auch Monomere f) als Aufbaukomponenten auf, die sich jeweils an den Kettenenden befinden und diese abschließen.

Diese Aufbaukomponenten leiten sich zum einen von monofunktionellen, mit NCO-Gruppen reaktiven Verbindungen ab, wie Monoaminen, insbesondere mono-sekundären Aminen oder Monoalkoholen. Genannt seien hier beispielsweise Ethanol, n-Butanol, Ethylenglykol-monobutylether, 2-Ethylhexanol, 1-Octanol, 1-Dodecanol, 1-Hexadecanol, Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin und geeignete substituierte Derivate davon.

Da die Bausteine f) im Wesentlichen in den erfindungsgemäßen Polyurethanharnstofflösungen dazu verwendet werden, den NCO-Überschuss zu vernichten, hängt die erforderliche Menge im Wesentlichen von der Menge des NCO-Überschusses ab und kann nicht allgemein spezifiziert werden.

In einer bevorzugten Ausführungsform der vorliegenden Erfindung wird auf die Komponente f) verzichtet, so dass der erfindungswesentliche Polyurethanharnstoff nur die Bestandteile a) bis d) und gegebenenfalls die Komponente e) umfasst.

Bei der Herstellung der erfindungswesentlichen Polyurethanharnstoffe werden die oben näher beschriebenen Aufbaukomponenten im Allgemeinen so umgesetzt, dass zunächst ein harnstoffgruppenfreies, isocyanatfunktionelles Prepolymer durch Umsetzung der Bestandteile a), b), c) und gegebenenfalls e) hergestellt wird, wobei das Stoffinengenverhältnis von Isocyanatgruppen zu mit Isocyanat reaktiven Gruppen vorzugsweise 0,8 bis 4,0, besonders bevorzugt 0,9 bis 3,8 insbesondere 1,0 bis 3,5 beträgt.

In einer alternativen Ausführungsform kann auch erst der Bestandteil a) separat mit dem Isocyanat b) umgesetzt werden. Danach kann dann die Zugabe der Bestandteile c) und e) und deren Umsetzung erfolgen. Anschließend werden im Allgemeinen die verbliebenen Isocyanat-Gruppen vor, während oder nach dem Dispergieren in Wasser aminofunktionell kettenverlängert oder terminiert, wobei das Äquivalentverhältnis von isocyanatreaktiven Gruppen der zur Kettenverlängerung eingesetzten Verbindungen zu freien Isocyanat-Gruppen des Prepolymers vorzugsweise zwischen 40 bis 150 %, besonders bevorzugt zwischen 50 bis 120 %, insbesondere zwischen 60 bis 120 %, liegt (Bestandteil d)).

Die erfindungsgemäßen Polyurethandispersionen werden dabei vorzugsweise nach dem sogenannten Aceton-Verfahren hergestellt. Für die Herstellung der Polyurethandispersion nach diesem Aceton-Verfahren werden üblicherweise die Bestandteile a), c) und e), die keine primären oder sekundären Aminogruppen aufweisen dürfen und die Polyisocyanatkomponente b) zur Herstellung eines isocyanatfunktionellen Polyurethan-Prepolymers ganz oder teilweise vorgelegt und gegebenenfalls mit einem mit Wasser mischbaren, aber gegenüber Isocyanatgruppen inerten Lösungsmittel verdünnt und auf Temperaturen im Bereich von 50 bis 120 °C aufgeheizt. Zur Beschleunigung der Isocyanatadditionsreaktion können die in der Polyurethan-Chemie bekannten Katalysatoren eingesetzt werden, beispielsweise Dibutylzinndilaurat. Bevorzugt ist die Synthese ohne Katalysator. Geeignete Lösungsmittel sind die üblichen aliphatischen, ketofunktionellen Lösemittel wie z.B. Aceton, Butanon, die nicht nur zu Beginn der Herstellung, sondern gegebenenfalls in Teilen auch später zugegeben werden können. Bevorzugt sind Aceton und Butanon. Andere Lösemittel wie z.B. Xylol, Toluol, Cyclohexan, Butylacetat, Methoxypropylacetat, Lösemittel mit Ether- oder Estereinheiten können ebenfalls eingesetzt und ganz oder teilweise abdestilliert werden oder vollständig in der Dispersion verbleiben.

Anschließend werden die gegebenenfalls zu Beginn der Reaktion noch nicht zugegebenen Bestandteile von c) und e) zudosiert.

In einer bevorzugten Weise wird das Prepolymer ohne Lösungsmittelzusatz hergestellt und erst für die Kettenverlängerung mit einem geeigneten Lösungsmittel, vorzugsweise Aceton, verdünnt.

Die Umsetzung zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Anschluss wird in einem weiteren Verfahrensschritt, falls noch nicht oder nur teilweise geschehen, das erhaltene Prepolymer mit Hilfe von aliphatischen Ketonen wie Aceton oder Butanon gelöst.

Anschließend werden mögliche NH₂-, NH-funktionelle und/oder OH-funktionelle Komponenten mit den noch verbliebenen Isocyanatgruppen umgesetzt. Diese Kettenverlängerung/-terminierung kann dabei entweder in Lösungsmittel vor dem Dispergieren, während des Dispergierens oder in Wasser nach dem Dispergieren durchgeführt werden. Bevorzugt wird die Kettenverlängerung vor der Dispergierung in Wasser durchgeführt.

Werden zur Kettenverlängerung Verbindungen entsprechend der Definition von d) mit NH₂- oder NH-Gruppen eingesetzt, erfolgt die Kettenverlängerung der Prepolymere bevorzugt vor der Dispergierung.

Der Kettenverlängerungsgrad, also das Äquivalentverhältnis von NCO-reaktiven Gruppen der zur Kettenverlängerung eingesetzten Verbindungen zu freien NCO-Gruppen des Prepolymers liegt vorzugsweise zwischen 40 bis 150 %, besonders bevorzugt zwischen 50 bis 120 %, insbesondere zwischen 60 bis 120 %.

Die aminischen Komponenten d) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mitverwendet werden, so beträgt der Verdünnungsmittelgehalt bevorzugt 70 bis 95 Gew.-%.

Die Herstellung der Polyurethandispersion aus den Prepolymeren erfolgt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den Prepolymerlösungen gerührt. Bevorzugt wird das Wasser in das gelöste Prepolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Feststoffgehalt der Polyurethandispersion nach der Synthese liegt im Bereich von 20 bis 70 Gew.-%, bevorzugt 20 bis 65 Gew.-%. Für Beschichtungsversuche können diese Dispersionen beliebig mit Wasser verdünnt werden, um die Dicke der Beschichtung variabel einstellen zu können. Alle Konzentrationen von 1 bis 60 Gew.-% sind möglich, bevorzugt sind Konzentrationen im Bereich 1 bis 40 Gew.-%.

Dabei können beliebige Schichtdicken erreicht werden wie beispielsweise einige 100 nm bis hinauf zu einigen 100 µm, wobei im Rahmen der vorliegenden Erfindung auch höhere und geringere Dicken möglich sind.

Die erfindungsgemäßen Polyurethanharnstoffdispersionen können ferner für den jeweils angestrebten Einsatzzweck übliche Bestandteile und Additive enthalten.

Ein Beispiel hierfür sind pharmakologische Wirkstoffe, Arzneimittel und Additive, welche die Freisetzung von pharmakologischen Wirkstoffen fördern ("drug-eluting-Additive").

Pharmakologische Wirkstoffe bzw. Arzneimittel, welche in den erfindungsgemäßen Beschichtungen auf den medizinischen Geräten verwendet werden können und damit in den erfindungsgemäßen Lösungen enthalten sein können, sind beispielsweise thromboresistente Mittel, antibiotische Mittel, Antitumormittel, Wachtumshormone, Antivirusmittel, antiangiogene Mittel, angiogene Mittel, antimitotische Mittel, entzündungshemmende Mittel, Zellzyklus-regulierende Mittel, genetische Mittel, Hormone, sowie deren Homologe, Derivate, Fragmente, pharmazeutische Salze und Kombinationen davon.

Spezifische Beispiele solcher pharmakologischen Wirkstoffe bzw. Armeimittel schließen somit thromboresistente (nichtthrombogene) Mittel bzw. andere Mittel zur Unterdrückung einer akuten Thrombose, Stenose oder späten Re-Stenose der Arterien ein, beispielsweise Heparin, Streptokinase, Urokinase, Gewebe-Plasminogen-Aktivator, Anti-Thromboxan-B₂-Mittel; Anti-B-Thromoboglobulin, Prostaglandin-E, Aspirin, Dipyridimol, Anti-Thromboxan-A₂-Mittel, muriner monoklonaler Antikörper 7E3, Triazolopyrimidin, Ciprosten, Hirudin, Ticlopidin, Nicorandil usw. Ein Wachstumsfaktor kann als ebenfalls als ein Arzneimittel benutzt werden, um unterintimale fibromuskülare Hyperplasie an der arteriellen Stenosestelle zu unterdrücken, bzw. es kann jeder beliebige andere Hemmstoff des Zellwachstums an der Stenosestelle benutzt werden.

Der pharmakologische Wirkstoff bzw. das Arzneimittel kann auch aus einem Vasodilatator bestehen, um Vasospasmus entgegen zu wirken, zum Beispiel ein Antispasmusmittel wie Papaverin. Das Arzneimittel kann ein vasoaktives Mittel an sich sein, wie Calciumantagonisten, oder α- und β-adrenergische Agonisten oder Antagonisten. Zusätzlich kann das therapeutische Mittel ein biologisches Haftmittel wie Cyanoacrylat in medizinischer Qualität oder Fibrin, welche beispielsweise für das Ankleben einer Gewebeklappe an die Wand einer Koronararterie verwendet wird, sein.

Das therapeutische Mittel kann ferner ein antineoplastisches Mittel wie 5-Fluorouracil, vorzugsweise mit einem kontrollierenden freisetzenden Träger für das Mittel, sein (z.B. für die Anwendung eines fortdauernden kontrollierten freisetzenden antineoplastischen Mittels an einer Tumorstelle).

Das therapeutische Mittel kann ein Antibiotikum, vorzugsweise in Kombination mit einem kontrollierenden freisetzenden Träger für die fortdauernde Freisetzung aus der Beschichtung eines medizinischen Geräts an einen lokalisierten Infektionsherd innerhalb des Körpers, sein. Ähnlich kann das therapeutische Mittel Steroide für den Zweck des Unterdrückens einer Entzündung in lokalisiertem Gewebe oder aus anderen Gründen enthalten.

Spezifische Beispiele geeigneter Arzneimittel umfassen:
a) Heparin, Heparinsulfat, Hirudin, Hyaluronsäure, Chondroitinsulfat, Dermatansulfat, Keratansulfat, lytische Mittel, einschließlich Urokinase und Streptokinase, deren Homologe, Analoge, Fragmente, Derivate und pharmazeutische Salze davon;
b) antibiotische Mittel wie Penicilline, Cephalosporine, Vacomycine, Aminoglycoside, Quinolone, Polymyxine, Erythromycine; Tetracycline, Chloramphenicole, Clindamycine, Lincomycine, Sulfonamide, deren Homologe, Analoge, Derivate, pharmazeutische Salze und Mischungen davon;
c) Paclitaxel, Docetaxel, Immunsuppressiva wie Sirolimus oder Everolimus, Alkylierungsmittel einschließlich Mechlorethamin, Chlorambucil, Cyclophosphamid, Melphalan und Ifosfamid; Antimetaboliten einschließlich Methotrexat, 6-Mercaptopurin, 5-Fluorouracil und Cytarabin; Pflanzenalkoide einschließlich Vinblastin; Vincristin und Etoposid; Antibiotika einschließlich Doxorubicin, Daunomycin, Bleomycin und Mitomycin; Nitrosurea einschließlich Carmustin und Lomustin; anorganische Ionen einschließlich Cisplatin; biologische Reaktionsmodifikatoren einschließlich Interferon; angiostatinische Mittel und endostatinische Mittel; Enzyme einschließlich Asparaginase; und Hormone einschließlich Tamoxifen und Flutamid, deren Homologe, Analoge, Fragmente, Derivate, pharmazeutische Salze und Mischungen davon;
d) antivrale Mittel wie Amantadin, Rimantadin, Rabavirin, Idoxuridin, Vidarabin, Trifluridin, Acyclovir, Ganciclorir, Zidovudin, Phosphonoformate, Interferone, deren Homologe, Analoge, Fragmente, Derivate, pharmazeutische Salze und Mischungen davon; und
e) entzündungshemmende Mittel wie beispielsweise Ibuprofen, Dexamethason oder Methylprednisolon.

Zur Erzeugung von Oberflächen mit bewuchshemmenden Eigenschaften können die erfindungsgemäßen Beschichtungszusammensetzungen die aus dem Stand der Technik bekannten bewuchshemmenden Wirkstoffe enthalten. Deren Anwesenheit verstärkt in der Regel die bereits hervorragenden bewuchshemmenden Eigenschaften der mit den findungsgemäßen Beschichtungszusammensetzungen selbst erzeugten Oberflächen.

Weitere Zusätze wie beispielsweise Antioxidantien, Pigmente, Griffhilfsmittel, Farbstoffe, Mattierungsmittel, UV-Stabilisatoren, Lichtstabilisatoren, Hydrophobierungsmittel, Puffersubstanzen, Verlaufshilfsmittel und/oder Verdicker zur Viskositätseinstellung können verwendet werden.

Die erfindungsgemäßen Polyurethanharnstoffdispersionen können dazu verwendet werden, eine Beschichtung zum Beispiel auf einem medizinischen Gerät zu bilden.

Die Bezeichnung "medizinisches Gerät" ist im Rahmen der vorliegenden Erfindung breit zu verstehen. Geeignete, nicht einschränkende Beispiele für medizinische Geräte (einschließlich Instrumente) sind Kontaktlinsen; Kanülen; Kathetern, zum Beispiel urologische Kathetern wie Blasenkathetern oder Hamleiterkathetem; zentralvenöse Katheter; venöse Kathetern oder Einlass- bzw. Auslass-Kathetern; Dilationsballons; Kathetern für die Angioplastie und die Biopsie; Kathetern, die für das Einbringen eines Stents, eines Propf- oder eines Kavafilters verwendet werden; Ballonkathetern oder andere dehnbare medizinische Geräte; Endoskope; Larnygoskope; Trachealgeräte wie Endotrachealschläuche, Atemgeräte und andere Trachealabsauggeräte; bronchoalveolare Spülungskathetern; Kathetern, die bei der Koronarangioplastie verwendet werden; Führungsstäbe, Einführer und Ähnliches; Gefäßpfropfen; Schrittmacherteile; Cochleaimplantate; Zahnimplantatschläuche für die Nahrungszufuhr, Dränageschläuche; und Führungsdrähte.

Darüber hinaus können die erfindungsgemäßen Dispersionen zur Herstellung von Schutzbeschichtungen, zum Beispiel für Handschuhe, Stents und andere Implantate; extrakorporale (außerkörperliche) Blutschläuche (Blutführungsrohre); Membrane, zum Beispiel für die Dialyse; Blutfilter; Geräte für die Kreislaufunterstützung; Verbandsmaterial für die Wundpflege; Hambeutel und Stomabeutel verwendet werden. Eingeschlossen sind auch Implantate, die ein medizinisch wirksames Mittel enthalten, wie medizinisch wirksame Mitteln für Stents oder für Ballonoberflächen oder für Kontrazeptiva.

Üblicherweise wird das medizinisches Gerät aus Kathetern, Endoskopen, Laryngoskopen, Endotrachealschläuchen, Ernährungsschläuchen, Führungsstäben, Stents, und andere Implantate gebildet.

Beschichtungen ausgehend von den erfindungsgemäßen Dispersionen sind gerade deshalb besonders vorteilhaft für medizinische Anwendungen, da keine organischen Lösemittelreste enthalten und damit toxisch im Allgemeinen unbedenklich sind, und gleichzeitig zu einer ausgeprägteren Hydrophilie führen, was sich beispielsweise an einem geringen Kontaktwinkel ausmachen lässt.

Zusätzlich zu den hydrophilen Eigenschaften der Verbesserung der Gleitfähigkeit zeichnen sich die erfindungsgemäß vorgesehenen Beschichtungszusammensetzungen auch durch eine hohe Blutverträglichkeit aus. Hierdurch ist auch ein Arbeiten mit diesen Beschichtungen besonders im Blutkontakt vorteilhaft. Die Materialien zeigen im Vergleich zu Polymeren des Standes der Technik eine reduzierte Gerinnungsneigung im Blutkontakt.

Wirkstofffreisetzende Systeme basierend auf den erfindungsgemäßen hydrophilen Beschichtungsmaterialien sind auch außerhalb der Medizintechnik denkbar, beispielsweise für Anwendungen im Pflanzenschutz als Trägermaterial für Wirkstoffe. Die gesamte Beschichtung kann dann als wirkstofffreisetzendes System betrachtet werden und beispielsweise zur Beschichtung von Saatgut (Samenkörner) eingesetzt werden. Durch die hydrophilen Eigenschaften der Beschichtung kann der enthaltene Wirkstoff im feuchten Erdreich austreten und seine bestimmungsgemäße Wirkung entfalten ohne dass die Keimfähigkeit des Saatgutes beeinträchtigt wird. Im trockenen Zustand bindet das Beschichtungsmittel den Wirkstoff jedoch sicher an das Saatgut, so dass beispielsweise beim Einschießen des Samenkorns mit der Ausbringmaschine in den Boden der Wirkstoff nicht abgelöst wird, wodurch er unerwünschte Wirkungen z.B. auf die vorhandene Fauna entfalten könnte (Bienengefährdung durch Insektizide, die an sich den Insektenbefall des Samenkorns im Boden verhindern sollen).

Über die Anwendung als Beschichtung für medizinische Geräte hinaus können die erfindungsgemäßen Polyurethandispersionen auch für weitere technische Anwendungen im nicht-medizinischen Bereich benutzt werden.

So dienen die erfindungsgemäße Polyurethandispersionen zu Herstellung von Beschichtungen als Schutz von Oberflächen vor Beschlagen mit Feuchtigkeit, zur Herstellung von leicht zu reinigenden oder von selbstreinigenden Oberflächen. Diese hydrophilen Beschichtungen verringern auch die Aufnahme von Schmutz und verhindern die Bildung von Wasserflecken. Denkbare Anwendungen im Außenbereich sind beispielsweise Fensterscheiben und Dachluken, Glasfassaden oder Plexiglasdächer. Im Innenbereich können solche Materialien für die Beschichtung von Oberflächen im Sanitärbereich benutzt werden. Weitere Anwendungen sind die Beschichtung von Brillengläsern oder von Verpackungsmaterialien wie Lebensmittelverpackungen zur Vermeidung von Feuchtigkeitsbeschlag oder Tropfenbildung durch kondensiertes Wasser.

Die erfmdungsgemäße Polyurethandispersionen eignen sich ebenso zur Ausrüstung von Oberflächen im Kontakt mit Wasser zur Verminderung des Bewuchses. Diesen Effekt nennt man auch Antifoulingeffekt. Eine sehr wichtige Anwendung dieses Antifoulingeffektes ist im Bereich der Unterwasseranstriche von Schiffsrümpfen. Schiffsrümpfe ohne Antifoulingausrüstung werden sehr schnell von Meeresorganismen bewachsen, was durch erhöhte Reibung zu einer Verringerung der möglichen Geschwindigkeit und einem höheren Treibstoffverbrauch führt. Die erfindungsgemäßen Beschichtungsmaterialien reduzieren oder verhindern den Bewuchs mit Meeresorganismen und verhindern die oben beschriebenen Nachteile dieses Bewuchses. Weitere Anwendungen im Bereich der Antifoulingbeschichtungen sind Artikel für die Fischerei wie Fischernetze sowie alle metallischen Substrate im Unterwassereinsatz wie Rohrleitungen, Bohrinseln, Schleusenkammern und -tore etc. Schiffsrümpfe, die mit den erfindungsgemäßen Beschichtungsmaterialien erzeugte Oberflächen insbesondere unterhalb der Wasserlinie aufweisen, besitzen auch einen reduzierten Reibungswiderstand, so dass derartig ausgerüstete Schiffe entweder einen reduzierten Brennstoff-Verbrauch aufweisen oder höhere Geschwindigkeiten erreichen. Dies ist insbesondere im Sportbootbereich und Yachtbau von Interesse.

Ein weiteres wichtiges Anwendungsgebiet der obengenannten hydrophilen Beschichtungsmaterialien ist die Druckindustrie. Hydrophobe Oberflächen können durch die erfindungsgemäßen Beschichtungen hydrophilisiert werden und sind dadurch mit polaren Druckfarben bedruckbar bzw. können mittels Tintenstrahltechnik aufgebracht werden.

Ein weiterer Anwendungsbereich der erfindungsgemäßen hydrophilen Beschichtungen sind Formulierungen für kosmetischen Anwendungen.

Beschichtungen aus den erfindungsgemäßen Polyurethandispersionen können mittels verschiedener Verfahren aufgebracht werden. Geeignete Beschichtungstechniken sind hierfür beispielsweise Rakeln, Drucken, Transferbeschichten, Sprühen, Spincoating oder Tauchen.

Beschichtet werden können vielerlei Substrate wie Metalle, Textilien, Keramiken und Kunststoffe. Bevorzugt ist die Beschichtung von medizinischen Geräten, die aus Kunststoff oder Metallen gefertigt sind. Als Metalle können beispielsweise genannt werden: Medizinischer Edelstahl und Nickel-Titan-Legierungen. Es sind viele Polymermaterialien denkbar, aus denen das medizinische Geräte aufgebaut sein kann, beispielsweise Polyamid; Polystyrol; Polycarbonat; Polyether; Polyester; Polyvinylacetat; natürliche und synthetische Kautschuke; Blockcopolymere aus Styrol und ungesättigten Verbindungen wie Ethylen, Butylen und Isopren; Polyethylen oder Copolymeren aus Polyethylen und Polypropylen; Silikon; Polyvinylchlorid (PVC) und Polyurethanen. Zur besseren Haftung des hydrophilen Polyurethane auf dem medizinischen Gerät können als Untergrund vor dem Auftragen dieser hydrophilen Beschichtungsmaterialien noch weitere geeignete Beschichtungen aufgetragen werden.

### Beispiele

Die Ermittlung des NCO-Gehaltes der in den Beispielen und Vergleichsbeispielen beschriebenen Harze erfolgte durch Titration gemäß DIN EN ISO 11909.

Die Bestimmung der Festkörpergehalte erfolgte nach DIN-EN ISO 3251. Es wurden 1 g Polyurethandispersion bei 115 °C bis zur Gewichtskonstanz (15-20 min) mittels eines Infrarottrockners getrocknet.

Die Messung der mittleren Teilchengrößen der Polyurethandispersionen erfolgt mit Hilfe des High Performance Particle Sizer (HPPS 3.3) der Firma Malvem Instruments.

Die Zugfestigkeiten wurden nach DIN 53504 bestimmt.

Die in % angegebenen Mengenangaben verstehen sich, wenn nicht anders vermerkt, als Gew.-% und beziehen sich auf die erhaltene wässrige Dispersion.

### Verwendete Substanzen und Abkürzungen:

| | |
|---|---|
| Desmophen C2200: | Polycarbonatpolyol, OH-Zahl 56 mg KOH/g, zahlenmittleres Molekulargewicht 2000 g/mol (Bayer, MaterialScience AG, Leverkusen, DE) |
| | |
| Desmophen C 1200: | Polycarbonatpolyol, OH-Zahl 56 mg KOH/g, zahlenmittleres Molekulargewicht 2000 g/mol (Bayer MaterialScience AG, Leverkusen, DE) |
| | |
| Desmophen XP 2613 | Polycarbonatpolyol, OH-Zahl 56 mg KOH/g, zahlenmittleres Molekulargewicht 2000 g/mol (Bayer MaterialScience AG, Leverkusen, DE) |
| | |
| Polyether LB 25: | (monofunktioneller Polyether auf Ethylenoxid/Propylenoxidbasis zahlenmittleres Molekulargewicht 2250 g/mol, OH-Zahl 25 mg KOH/g (Bayer MaterialScience AG, Leverkusen, DE) |
| | |
| TCD-Alkohol DM | 3(4),8(9)-Bis(hydroxymethyl)-tricyclo(5.2.1.0/2.6)decan/ Tricyclodecandimethanol der Firma Celanese Chemicals, Dallas, USA |

### Beispiel 1:

### Herstellung eines cycloaliphatischen Polycarbonatdiols auf Basis TCD-Alkohol DM mit einem zahlenmittleren Molekulargewicht von 1.300 g/mol

In einem 16 1 Druckreaktor mit Destillationsaufsatz, Rührer und Vorlage wurden 5.436 g TCD Alkohol DM mit 1,2 g Yttrium(III)acetylacetonat sowie 3.810 g Dimethylcarbonat bei 80 °C vorgelegt. Anschließend wurde unter Stickstoffatmosphäre das Reaktionsgemisch in 2 h auf 135 °C aufgeheizt und dort unter Rühren 24 h gehalten wobei der Druck auf 6,3 bar (absolut) anstieg. Danach wurde auf 60 °C abgekühlt und belüftet. Dann wurde das Spaltprodukt Methanol im Gemisch mit Dimethylcarbonat per Destillation entfernt, wobei die Temperatur schrittweise auf 150 °C erhöht wurde. Es wurde dann noch 4 Stunden bei 150 °C gerührt, anschließend auf 180 °C aufgeheizt und dann noch 4 h bei 180 °C gerührt. Dann wurde die Temperatur auf 90 °C reduziert und ein Stickstoffstrom (5 1/h) durch das Reaktionsgemisch hindurchgeleitet, während der Druck auf 20 mbar abgesenkt wurde. Danach wurde die Temperatur binnen 4 h auf 180 °C erhöht und dort 6 h gehalten. Dabei erfolgte die weitere Entfernung von Methanol im Gemisch mit Dimethylcarbonat aus dem Reaktionsgemisch.

Nach Belüftung und Abkühlung des Reaktionsansatzes auf Raumtemperatur, wurde ein gelbliches, festes Polycarbonatdiol mit folgenden Kennzahlen erhalten:
Mₙ = 1.290 g/mol; OH-Zahl = 87 mg KOH/g;

### Beispiel 2:

### Herstellung eines cycloaliphatischen Polycarbonatdiols auf Basis TCD-Alkohol DM mit einem zahlenmittleren Molekulargewicht von ca. 500 g/mol

### Vorgehen wie in Beispiel 1, wobei 7790 g TCD-Alkohol DM, 1,68 g Yttrium(III)acetylacetonat und 3096 g Dimethylcarbonat verwendet wurden.

Man erhielt ein gelbliches, hochviskoses Polycarbonatdiol mit folgenden Kennzahlen: Mₙ = 496 g/mol; OH-Zahl = 226 mg KOH/g; Viskosität bei 75 °C = 138.400 mPas.

### Beispiel 3:

### Herstellung eines (cyclo)aliphatischen Polycarbonatdiols auf Basis TCD-Alkohol DM und 1,4-Butandiol mit einem zahlenmittleren Molekulargewicht von ca. 1.000 g/mol

Vorgehen wie in Beispiel 1, wobei 5951 g TCD-Alkohol DM, 2732 g 1,4-Butandiol, 2,0 g Yttrium(III)acetylacetonat und 6842 g Dimethylcarbonat verwendet wurden.

Man erhielt ein farbloses, Polycarbonatdiol mit folgenden Kennzahlen: Mₙ = 943 g/mol; OH-Zahl = 119 mg KOH/g; Viskosität bei 75 °C = 15.130 mPas.

### Beispiel 4: (Vergleich)

277,2 g Desmophen C 2200, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isoeyanatocyclohexyl)methan (H₁₂MDI) und danach 11,9 g Isophorondiisocyanat. Man erwärmte auf 110 °C. Nach 16 h war der theoretische NCO Wert von 2,4% erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 40,7 % und einer mittleren Teilchengröße von 136 nm erhalten.

### Beispiel 5: (erfindungsgemäß)

208,0 g Desmophen C 2200, 45,2 g Polycarbonatdiol des Beispiels 1, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 16 h war der theoretische NCO Wert von 2,6 % erreicht. Das fertige Prepolymer wurde bei 50 °C in 700 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 550 g Wasser dispergiert. Es folgte die Entfernung des Lösungsmittels durch Destillation im Vakuum. Es wurde eine lagerstabile Polyurethandispersion mit einem Festkörpergehalt von 39,0 % und einer mittleren Teilchengröße von 131 nm erhalten.

### Beispiel 6: (erfindungsgemäß)

138,6 g Desmophen C 2200, 90,1 g Polycarbonatdiol des Beispiels 1, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 2 h 45 min war der theoretische NCO Wert von 2,8 % erreicht. Das fertige Prepolymer wurde bei 50 °C in 700 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 550 g Wasser dispergiert. Es folgte die Entfernung des Lösungsmittels durch Destillation im Vakuum. Es wurde eine lagerstabile Polyurethandispersion mit einem Festkörpergehalt von 39,6 % und einer mittleren Teilchengröße von 157 nm erhalten.

### Beispiel 7: (erfindungsgemäß)

184,8 g Desmophen C 2200, 23,1 g Polycarbonatdiol des Beispiels 2, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 21 h war der theoretische NCO Wert von 2,9 % erreicht. Das fertige Prepolymer wurde bei 50 °C in 650 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 490 g Wasser dispergiert. Es folgte die Entfernung des Lösungsmittels durch Destillation im Vakuum. Es wurde eine lagerstabile Polyurethandispersion mit einem Festkörpergehalt von 40,3 % und einer mittleren Teilchengröße von 117 nm erhalten.

### Beispiel 8: (erfindungsgemäß)

138,6 g Desmophen C 2200, 34,7 g Polycarbonatdiol des Beispiels 2, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 18 h war der theoretische NCO Wert von 3,3 % erreicht. Das fertige Prepolymer wurde bei 50 °C in 650 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 450 g Wasser dispergiert. Es folgte die Entfernung des Lösungsmittels durch Destillation im Vakuum. Es wurde eine lagerstabile Polyurethandispersion mit einem Festkörpergehalt von 40,5 % und einer mittleren Teilchengröße von 151 nm erhalten.

### Beispiel 9: (erfindungsgemäß)

138,6 g Desmophen C 2200, 69,3 g Polycarbonatdiol des Beispiels 3, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 2 h 15 min war der theoretische NCO Wert von 2,9 % erreicht. Das fertige Prepolymer wurde bei 50 °C in 650 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 520 g Wasser dispergiert. Es folgte die Entfernung des Lösungsmittels durch Destillation im Vakuum. Es wurde eine lagerstabile Polyurethandispersion mit einem Festkörpergehalt von 38,0 % und einer mittleren Teilchengröße von 190 nm erhalten.

### Beispiel 10: Kontaktwinkel und 100 %-Module von Vergleichsbeispiel 4 gegen erfindungsgemäße Beispiele 5-9

### 1. Herstellung der Beschichtungen für die Messung des statischen Kontaktwinkels

Die Beschichtungen zur Messung des statischen Kontaktwinkels wurden auf Glasobjektträger der Größe 25x75 mm mit Hilfe eines Spincoaters (RC5 Gyrset 5, Karl Süss, Garching, Deutschland) hergestellt. Ein Objektträger wurde hierzu auf dem Probenteller des Spincoaters eingespannt und mit ca. 2,5 - 3 g wässriger unverdünnter Polyurethandispersion homogen bedeckt. Durch Rotation des Probentellers für 20 sec bei 1300 Umdrehungen pro Minute erhielt man eine homogene Beschichtung, die 15 min bei 100 °C und danach 24 h bei 50 °C getrocknet wurde. Die erhaltenen beschichteten Objektträger wurden direkt einer Kontaktwinkelmessung unterzogen.

Von den erhaltenen Beschichtungen auf den Objektträgern wird eine statische Kontaktwinkelmessung durchgeführt. Mittels des Video- Randwinkelmessgerätes OCA20 der Firma Dataphysics mit rechnergesteuerten Spritzen wird auf das Muster 10 Tropfen Milliporewasser aufgesetzt und deren statischer Benetzungsrandwinkel gemessen. Zuvor wird mittels eines Antistatikföns die statische Aufladung (falls vorhanden) auf der Probenoberfläche entfernt.

### 2. Herstellung der Beschichtungen für die Messung des 100%-Moduls

Auf Trennpapier werden mit einem 200µm-Rakel Schichten hergestellt, die 15 min bei 100 °C getrocknet werden. Danach erfolgt Trocknung für 15 min bei 100 °C. Ausgestanzte Formkörper werden nach DIN 53504 untersucht.

Die Beschichtungen wurden auf Trennpapier mit einem 200 µm-Rakel aufgetragen. Vor der Filmherstellung werden die wässrigen Dispersionen mit 2 Gew% eines Verdickers (Borchi Gel A LA, Fa. Borchers, Langenfeld, Deutschland) versetzt und 30 min bei RT durch Rühren homogenisiert. Die feuchten Filme wurden 15 min bei 100 °C getrocknet.

Die Untersuchungen wurden gemäß DIN 53504 durchgeführt.

### 3. Untersuchungsergebnisse

**Tabelle 1: Kontaktwinkel und 100%-Module der Filme aus Materialien der Beispiele 4-9**

| Beispiel-Nr: | Kontaktwinkel (°) | 100%-Modul (N/mm²) |
|---|---|---|
| Vergleichsbeispiel 4 | 10 | 2,6 |
| Beispiel 5 | 12 | 3,0 |
| Beispiel 6 | 17 | 6,7 |
| Beispiel 7 | 16 | 3,6 |
| Beispiel 8 | 27 | 7,2 |
| Beispiel 9 | 16 | 5,4 |

Die erfindungsgemäßen Beispiele 5 bis 9 unterscheiden sich dadurch, dass im Vergleich zum Vergleichsbeispiel 4 ein Teil des Polycarbonatdiols Desmophen C2200 durch ein neues erfindungsgemäßes Polycarbonatdiol ersetzt wurde. Die Materialien zeigen als Beschichtung ähnlich hydrophile Eigenschaften wie das Vergleichsbeispiel 4, immer kleinere Kontaktwinkel als 30 °. Die 100%-Module sind alle höher als dasjenige des Vergleichsbeispiels 4.

### Beispiel 11: (Vergleich)

282,1 g Desmophen C 2200, 22,0 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 21,5 h war der theoretische NCO Wert von 2,4 % erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 41,7 % und einer mittleren Teilchengröße von 207 nm erhalten.

### Beispiel 12: (erfindungsgemäß)

141,2 g Desmophen C 2200, 35,3 g des Polycarbonatdiols des Beispiels 2, 22,0 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 18 h war der theoretische NCO Wert von 3,4 % erreicht. Das fertige Prepolymer wurde bei 50 °C in 600 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 400 g Wasser dispergiert. Es folgte die Entfernung des Lösungsmittels durch Destillation im Vakuum. Es wurde eine lagerstabile Polyurethandispersion mit einem Festkörpergehalt von 41,6 % und einer mittleren Teilchengröße von 219 nm erhalten.

### Beispiel 13: Kontaktwinkel und 100%-Module von Vergleichsbeispiel 11 gegen erfindungsgemäßes Beispiel 12

Die Herstellung der Beschichtungen sowie die Ermittlung der Kontaktwinkel und 100%-Module erfolgt wie im Beispiel 10 beschrieben.

**Tabelle 2: Kontaktwinkel und 100%-Module der Filme aus Materialien der Beispiele 12 und 13**

| Beispiel-Nr. | Kontaktwinkel (°) | 100%-Modul (N/mm²) |
|---|---|---|
| Vergleichsbeispiel 11 | 24 | 3,3 |
| Beispiel 12 | 36 | 9,2 |

Das erfindungsgemäße Beispiel 12 enthält im Vergleich zum Vergleichsbeispiel 11 Anteile eines erfindungsgemäßen Polycarbonatdiols. Die Oberfläche der Beschichtung ist nach wie vor sehr hydrophil, während sich das 100%-Modul sich fast verdreifacht.

### Beispiel 14: (Vergleich)

282,1 g Desmophen XP 2613, 22,0 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 70 min war der theoretische NCO-Wert von 2,5 % erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 38,3 % und einer mittleren Teilchengröße von 215 nm erhalten.

### Beispiel 15: (erfindungsgemäß)

141,2 g Desmophen XP 2613, 91,8 g des Polycarbonatdiols des Beispiels 1, 22,0 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 60 min war der theoretische NCO-Wert erreicht. Das fertige Prepolymer wurde bei 50 °C in 650 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 530 g Wasser dispergiert. Es folgte die Entfernung des Lösungsmittels durch Destillation im Vakuum. Es wurde eine lagerstabile Polyurethandispersion mit einem Festkörpergehalt von 38,2 % und einer mittleren Teilchengröße von 327 nm erhalten.

### Beispiel 16: Kontaktwinkel und 100%-Module von Vergleichsbeispiel 14 gegen erfindungsgemäßes Beispiel 15

Die Herstellung der Beschichtungen sowie die Ermittlung der Kontaktwinkel und 100%-Module erfolgt wie im Beispiel 10 beschrieben.

**Tabelle 3: Kontaktwinkel und 100%-Module der Filme aus Materialien der Beispiele 14 und 15**

| Beispiel-Nr. | Kontaktwinkel (°) | 100%-Modul (N/mm²) |
|---|---|---|
| Vergleichsbeispiel 14 | 41 | 3,0 |
| Beispiel 15 | 41 | 12,3 |

Das erfindungsgemäße Beispiel 15 enthält im Vergleich zum Vergleichsbeispiel 14 Anteile eines erfindungsgemäßen Polycarbonatdiols. Der Kontaktwinkel der Beschichtung änden sich fast gar nicht, während sich das 100%-Modul vervierfacht.

### Beispiel 17: (Vergleich)

269,8 g Desmophen C 2200, 49,7 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 21 h war der theoretische NCO-Wert von 2,4 % erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 41,3 % und einer mittleren Teilchengröße von 109 nm erhalten.

### Beispiel 18: (erfindungsgemäß)

135,0 g Desmophen C2200, 33,8 g des Polycarbonatdiols des Beispiels 2, 49,7 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 20 h war der theoretische NCO-Wert erreicht. Das fertige Prepolymer wurde bei 50 °C in 590 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 33,7 % und einer mittleren Teilchengröße von 83 nm erhalten.

### Beispiel 19: Kontaktwinkel und 100%-Module von Vergleichsbeispiel 17 gegen erfindungsgemäßes Beispiel 18

Die Herstellung der Beschichtungen sowie die Ermittlung der Kontaktwinkel und 100%-Module erfolgt wie im Beispiel 10 beschrieben.

**Tabelle 4: Kontaktwinkel und 100%-Module der Filme aus Materialien der Beispiele 17 und 18**

| Beispiel-Nr. | Kontaktwinkel (°) | 100%-Modul (N/mm²) |
|---|---|---|
| Vergleichsbeispiel 17 | 11 | 1,9 |
| Beispiel 18 | 9 | 6,0 |

Das erfindungsgemäße Beispiel 17 enthält im Vergleich zum Vergleichsbeispiel 18 Anteile eines erfindungsgemäßen Polycarbonatdiols. Der Kontaktwinkel der Beschichtung ändert sich fast gar nicht, während sich das 100%-Modul verdreifacht.

### Beispiel 20: (Vergleich)

277,2 g Desmophen C 2200, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 75 min war der theoretische NCO-Wert von 2,4 % erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 39,9 % und einer mittleren Teilchengröße von 169 nm erhalten.

### Beispiel 21: (erfindungsgemäß)

138,6 g Desmophen C2200, 34,7 g des Polycarbonatdiols des Beispiels 2, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 75 min war der theoretische NCO-Wert erreicht. Das fertige Prepolymer wurde bei 50 °C in 650 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 450 g Wasser dispergiert. Es folgte die Entfernung des Lösungsmittels durch Destillation im Vakuum. Es wurde eine lagerstabile Polyurethandispersion mit einem Festkörpergehalt von 40,0 % und einer mittleren Teilchengröße von 167 nm erhalten.

### Beispiel 22: Kontaktwinkel und 100%-Module von Vergleichsbeispiel 20 gegen erfindungsgemäßes Beispiel 21

Die Herstellung der Beschichtungen sowie die Ermittlung der Kontaktwinkel und 100%-Module erfolgt wie im Beispiel 10 beschrieben.

**Tabelle 5: Kontaktwinkel und 100%-Module der Filme aus Materialien der Beispiele 20 und 21**

| Beispiel-Nr. | Kontaktwinkel (°) | 100%-Modul (N/mm²) |
|---|---|---|
| Vergleichsbeispiel 20 | 14 | 1,6 |
| Beispiel 21 | 16 | 6,1 |

Das erfindungsgemäße Beispiel 21 enthält im Vergleich zum Vergleichsbeispiel 20 Anteile eines erfindungsgemäßen Polycarbonatdiols. Der Kontaktwinkel der Beschichtung ändert sich fast gar nicht, während sich das 100%-Modul fast vervierfacht.

## Patentansprüche

1. Polyurethanharnstoff-Dispersionen umfassend Polyurethanharnstoffe, die
(1) mit wenigstens einer polyethylenoxid- und polypropylenoxidbasierten Copolymereinheit terminiert sind, und
(2) Polycarbonatpolyol-basierte Einheiten gemäß Formel (I) umfassen

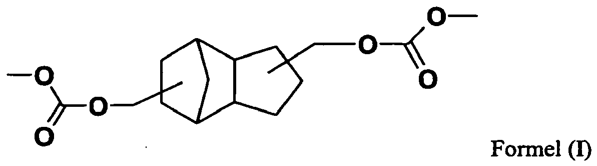

2. Polyurethanharnstoff-Dispersionen gemäß Anspruch 1, **dadurch gekennzeichnet, dass** die darin enthaltenen Polyurethanharnstoffe frei von ionischen oder ionogenen Gruppen sind.

3. Polyurethanharnstoff-Dispersionen gemäß Anspruch 1 oder 2, **dadurch gekennzeichnet, dass** die darin enthaltenen Polyurethanharnstoffe auf einer Polycarbonatpolyolkomponente basieren, die eine mittlere Hydroxylfunktionalität von bevorzugt 1,7 bis 2,3 aufweist.

4. Polyurethanharnstoff-Dispersionen gemäß Anspruch 3, **dadurch gekennzeichnet, dass** Polycarbonatpolyolkomponente Polycarbonatpolyole a1) aufweist, die durch Umsetzung von Kohlensäurederivaten mit difunktionellen Alkoholen der Formel (II) 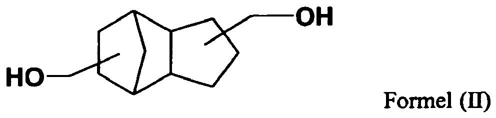 erhalten werden.

5. Polyurethanharnstoff-Dispersionen gemäß Anspruch 4, **dadurch gekennzeichnet, dass** Polycarbonatpolyolkomponente neben Polycarbonatpolyolen a1) auch weitere Polycarbonatpolyolen a2) aufweist.

6. Polyurethanharnstoff-Dispersionen gemäß Anspruch 5, **dadurch gekennzeichnet, dass** es sich bei den Polycarbonatpolyolen a2) um Verbindungen mit mittleren Hydroxylfunktionalitäten von 1,7 bis 2,3 und durch die OH-Zahl bestimmte Molekulargewichte von 400 bis 6000 g/mol auf Basis von Hexandiol-1,6, Butandiol-1,4 oder deren Mischungen handelt.

7. Polyurethanharnstoff-Dispersionen gemäß einem der Ansprüche 1 bis 6, **dadurch gekennzeichnet, dass** die zur Terminierung eingesetzte Copolymereinheit aus Polyethylenoxid und Polypropylenoxid auf einem monohydroxyfunktionellen gemischten Polyalkylenoxidpolyether aus mindestens 40 mol-% Ethylenoxid- und maximal 60 mol-% Propylenoxideinheiten bezogen auf den Gesamtanteil an Alkylenoxideinheiten basiert mit einem zahlenmittleren Molekulargewicht von 500 g/mol bis 5000 g/mol.

8. Polyurethanharnstoff-Dispersionen gemäß einem der Ansprüche 1 bis 7, **dadurch gekennzeichnet, dass** darin enthaltenen Polyurethanharnstoffe zahlenmittlere Molekulargewichte von 5000 bis 100000 g/mol gemessen in Dimethylacetamid bei 30 °C aufweisen.

9. Polyurethanharnstoff-Dispersionen gemäß einem der Ansprüche 1 bis 8, **dadurch gekennzeichnet, dass** diese pharmakologische Wirkstoffe umfassen.

10. Verfahren zur Herstellung der Polyurethanharnstoff-Dispersionen gemäß einem der Ansprüche 1 bis 9, bei dem eine Polycarbonatpolyolkomponente a), mindestens eine Polyisocyanatkomponente b), mindestens eine Polyoxyalkylenetherkomponente c), mindestens eine Diamin-und/oder Aminoalkoholkomponente d) und gegebenenfalls eine weitere Polyolkomponente miteinander umgesetzt werden und eine Dispergierung in Wasser erfolgt.

11. Polyurethanharnstoffe erhältlich aus Polyurethanharnstoffdispersionen gemäß einem der Ansprüche 1 bis 9.

12. Beschichtungen erhältlich unter Verwendung von Polyurethanhamstoffen gemäß Anspruch 11.

13. Substrate beschichtet mit Beschichtungen gemäß Anspruch 12.

## Claims

1. Polyurethaneurea dispersions comprising polyurethaneureas which
(1) are terminated with at least one polyethylene oxide- and polypropylene oxide-based copolymer unit, and
(2) comprise polycarbonate polyol-based units of formula (I)

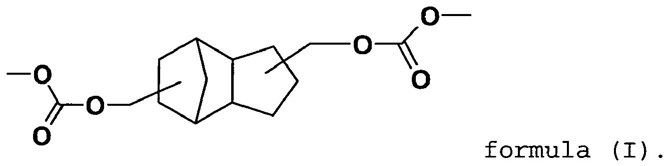

2. Polyurethaneurea dispersions according to Claim 1, **characterized in that** the polyurethaneureas present therein are free from ionic or ionogenic groups.

3. Polyurethaneurea dispersions according to Claim 1 or 2, **characterized in that** the polyurethaneureas present therein are based on a polycarbonate polyol component, which has an average hydroxyl functionality of preferably 1.7 to 2.3.

4. Polyurethaneurea dispersions according to Claim 3, **characterized in that** polycarbonate polyol component has polycarbonate polyols a1) which are obtained by reacting carbonic acid derivatives with difunctional alcohols of the formula (II)

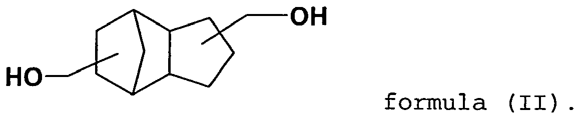

5. Polyurethaneurea dispersions according to Claim 4, **characterized in that** polycarbonate polyol component has not only polycarbonate polyols a1) but also further polycarbonate polyols a2).

6. Polyurethaneurea dispersions according to Claim 5, **characterized in that** the polycarbonate polyols a2) are compounds having average hydroxyl functionalities of 1.7 to 2.3 and molecular weights, as determined through the OH number, of 400 to 6000 g/mol, based on hexane-1,6-diol, butane-1,4-diol or mixtures thereof.

7. Polyurethaneurea dispersions according to any of Claims 1 to 6, **characterized in that** the copolymer unit of polyethylene oxide and polypropylene oxide that is used for termination is based on a monohydroxy-functional mixed polyalkylene oxide polyether of at least 40 mol% ethylene oxide units and not more than 60 mol% propylene oxide units, based on the total fraction of alkylene oxide units, with a number-average molecular weight of 500 g/mol to 5000 g/mol.

8. Polyurethaneurea dispersions according to any of Claims 1 to 7, **characterized in that** polyurethaneureas present therein have number-average molecular weights of 5000 to 100 000 g/mol as measured in dimethylacetamide at 30°C.

9. Polyurethaneurea dispersions according to any of Claims 1 to 8, **characterized in that** they comprise pharmacological actives.

10. Process for preparing the polyurethaneurea dispersions according to any of Claims 1 to 9, in which a polycarbonate polyol component a), at least one polyisocyanate component b), at least one polyoxyalkylene ether component c), at least one diamine and/or amino alcohol component d) and, if desired, a further polyol component are reacted with one another and dispersing in water takes place.

11. Polyurethaneureas obtainable from polyurethaneurea dispersions according to any of Claims 1 to 9.

12. Coatings obtainable using polyurethaneureas according to Claim 11.

13. Substrates coated with coatings according to Claim 12.

## Revendications

1. Dispersions de polyuréthane-urée comprenant des polyuréthane-urées qui
(1) sont terminées par au moins une unité de copolymère à base de poly(oxyde d'éthylène) et de poly(oxyde de propylène), et
(2) comprennent des unités à base de polycarbonatepolyol selon la formule (I)

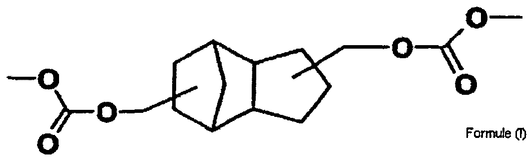

2. Dispersions de polyuréthane-urée selon la revendication 1, **caractérisées en ce que** les polyuréthane-urées qui y sont contenues sont exemptes de groupes ioniques ou ionogènes.

3. Dispersions de polyuréthane-urée selon la revendication 1 ou 2, **caractérisées en ce que** les polyuréthane-urées qui y sont contenues sont à base d'un composant de polycarbonatepolyol présentant une fonctionnalité hydroxyle moyenne de préférence de 1,7 à 2,3.

4. Dispersions de polyuréthane-urée selon la revendication 3, **caractérisées en ce que** le composant polycarbonatepolyol présente des polycarbonatepolyols a1) qui sont obtenus par transformation de dérivés d'acide carbonique avec des alcools difonctionnels de formule (II)

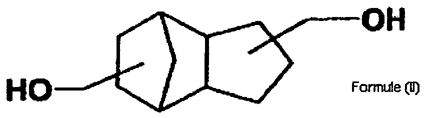

5. Dispersions de polyuréthane-urée selon la revendication 4, **caractérisées en ce que** le composant polycarbonatepolyol présente, outre les polycarbonatepolyols a1) également d'autres polycarbonatepolyols a2).

6. Dispersions de polyuréthane-urée selon la revendication 5, **caractérisées en ce qu'**il s'agit, pour les polycarbonatepolyols a2), de composés présentant des fonctionnalités hydroxyle moyennes de 1,7 à 2,3 et des poids moléculaires déterminés par l'indice OH de 400 à 6000 g/mole à base d'hexanediol-1,6, de butanediol-1,4 ou leurs mélanges.

7. Dispersions de polyuréthane-urée selon l'une quelconque des revendications 1 à 6, **caractérisées en ce que** l'unité copolymère de poly(oxyde d'éthylène) et de poly(oxyde de propylène) utilisée pour la terminaison est à base d'un poly(oxyde d'alkylène)-polyéther mixte à fonctionnalité monohydroxy constitué par au moins 40% en mole d'unités d'oxyde d'éthylène et au maximum 60% en mole d'unités d'oxyde de propylène par rapport à la proportion totale d'unités d'oxyde d'alkylène, présentant un poids moléculaire numérique moyen de 500 g/mole à 5000 g/mole.

8. Dispersions de polyuréthane-urée selon l'une quelconque des revendications 1 à 7, **caractérisées en ce que** les polyuréthane-urées qui y sont contenues présentent des poids moléculaires numériques moyens de 5000 à 100 000 g/mole, mesurés dans du diméthylacétamide à 30°C.

9. Dispersions de polyuréthane-urée selon l'une quelconque des revendications 1 à 8, **caractérisées en ce que** celles-ci comprennent des substances actives pharmacologiques.

10. Procédé pour la préparation des dispersions de polyuréthane-urée selon l'une quelconque des revendications 1 à 9, dans lequel un composant polycarbonatepolyol a), au moins un composant polyisocyanate b), au moins un composant polyoxyalkylène-éther c), au moins un composant diamine et/ou aminoalcool d) et le cas échéant un autre composant polyol sont transformés les uns avec les autres et une dispersion dans l'eau est réalisée.

11. Polyuréthane-urées pouvant être obtenues à partir de dispersions de polyuréthane-urées selon l'une quelconque des revendications 1 à 9.

12. Revêtements pouvant être obtenus en utilisant les polyuréthane-urées selon la revendication 11.

13. Substrats revêtus par des revêtements selon la revendication 12.
